# EUROPEAN PATENT SPECIFICATION

(11) **EP 2 974 655 B1**
(45) Date of publication and mention of the grant of the patent: **12.04.2017**
(21) Application number: 14002428.2
(22) Date of filing: 15.07.2014
(51) Int. Cl.: A61B 5/145, G01N 33/487, B25G 3/18, G01N 27/327

(54) **Handgrip test strip ejector**
Handgriffteststreifenauswerfer
Éjecteur de bande test de poignée

(43) Date of publication of application: 20.01.2016
(73) Proprietor: Roche Diabetes Care GmbH, 68305 Mannheim (DE); F. Hoffmann-La Roche AG, 4070 Basel (CH)
(72) Inventor: González Fernández, Andrea, 08028 Barcelona (ES); Ventayol Femenias, Bartomeu, 08028 Barcelona (ES)
(74) Representative: Riwotzki, Karsten

(56) References cited:
- US-A- 5 424 035
- US-A1- 2005 256 382
- US-A1- 2006 133 956

## Description

The present invention relates to handgrips configured to extract an analysis strip from a groove of an analyser.

### BACKGROUND ART

Analysers for analysing analysis strips are widely used. Some analysers have a groove for receiving an analysis strip. Such strips are configured to receive and retain a body fluid (e.g. blood) sample to be analysed. The groove into which the strip is to be inserted to carry out an analysis (of e.g. glucose in blood) is typically arranged on an end region of the analyser. Once the strip has been used (so that it still contains a body fluid sample), the strip has to be removed from the analyser. Removal of the strip by hand is not recommended in order to e.g. avoid risk of infection.

Analysers are known which do not have any kind of system for ejecting strips. In this case, an operator (doctor, nurse, etc.) can remove a used strip from the analyser by gripping and pulling the strip by hand. But, as commented before, this way of extracting the strip is not recommended at all.

Analysers are known having a system for automatically ejecting strips integrated within the analyser, in such a way that all the components of the analyser (including the ejection system) are covered and/or protected by a common casing of the analyser. These ejection systems are typically based on rather complex configurations/ mechanisms which are internal to the main casing of the analyser. Thus, if e.g. the ejection system needs to be repaired, the main casing of the analyser has to be dismounted, in which case other components, such as sensitive electronic elements, may be exposed to a certain risk of damage.

Other known ejection systems for an analyser are based on having a small wheel outside the main casing of the analyser and near the groove, the wheel being arranged to make a certain pressure on the strip (when it is inserted in the groove). In this case, the strip is ejected by an operator directly acting on (touching) the wheel to make it rotate, such that this rotation of the (pressing) wheel causes the strip to move out of the groove and, thus, to be ejected from the groove. A drawback of this approach may be that the operator has to act (by touching the wheel) on a position very close to the strip, so that the risk of infection may still be significant.

US 2005/256382 discloses a handgrip to extract an analysis strip from a groove of an analyser according to the preamble of claim1.

The present invention aims at providing a solution overcoming at least some of the previously commented problems/limitations.

### SUMMARY OF THE INVENTION

In a first aspect, the present invention provides a handgrip configured to extract an analysis strip from a groove of an analyser. The handgrip comprises a casing piece configured to be attached with the analyser, and a retaining piece having a retaining portion. The retaining piece is movable with respect to the casing piece in such a way that the retaining portion is movable in a first direction from a proximal position to a distal position relative to the casing piece. The retaining portion is configured to retain the analysis strip during at least part of its movement from the proximal to the distal position, and to avoid retaining the analysis strip at least when the retaining portion is in any of the proximal and distal positions.

With this handgrip suitably coupled to an analyser, an analysis strip may be easily inserted into a groove of the analyser when the retaining portion of the retaining piece is in its proximal position, since the retaining portion is configured to avoid retention of the strip when it is in the proximal position. This means that no opposition is presented by the retaining portion to the insertion of the strip into the groove in said proximal position.

Once inserted and processed by the analyser, the strip can be easily extracted from the groove by moving the retaining piece for causing movement of the retaining portion towards its distal position. As the retaining portion retains (or holds) the strip during at least part of said movement, pulling of the strip may be caused until it is taken out of the groove.

Once the strip has been taken out of the groove, then the retaining portion reaches its distal position and the strip is released because the retaining portion is configured to avoid retention of the strip also in said distal position. Ejection of the strip is therefore finally caused.

A handgrip for easily extracting an analysis strip from an analyser is therefore provided.

This proposed configuration is based on "externally" gripping and pulling the strip away from the groove (by the retaining portion). This approach may provide a significant versatility in terms of e.g. providing different possibilities with respect to manufacture and/or use of the handgrip. For example, the handgrip could be fabricated as a removable handgrip, such that the handgrip may be removed (uncoupled) from the analyser in a relatively easy manner. This way, a damaged handgrip may be easily replaced by a new or at least not damaged handgrip.

Alternatively, the handgrip could be fabricated integrated with the analyser, in such a way that the analyser and the handgrip may constitute a single product.

In any case, the handgrip, either a separate piece or a part of the analyser, may be repaired without necessity of dismounting the main casing of the analyser, such that other more sensitive components (such as e.g. electronic components) are not exposed to a certain risk of damage. Also, since the handgrip has a rather simple configuration and is external to the main casing of the analyser, the reparation of the handgrip may be easier and cheaper with respect to at least some prior art ejection systems.

Moreover, this approach may permit a variety of configurations in which the retaining piece may be moved (between the proximal and distal positions) by a corresponding operator (e.g. doctor, nurse, etc.) acting on positions significantly away from the strip. This way, the risk of infection may be significantly minimized.

In some examples, the retaining portion may comprise a pressing arrangement configured to exert pressure to the analysis strip, thereby causing the retention of the analysis strip, during the at least part of the movement of the retaining portion from the proximal to the distal position. This pressing arrangement may be further configured to avoid exerting pressure to the analysis strip, thereby causing the avoidance of retention of the analysis strip, at least when the retaining portion is in any of the proximal and distal positions.

In alternative configurations, one or more elastic (instead of pressing) arrangements may be used to achieve the same or similar functionalities (retention of the analysis strip and avoidance of retention of the analysis strip).

In particular examples, the pressing arrangement may comprise a first and a second pressing member facing each other and defining a path having a distance between them, said path being perpendicular to the first direction. These first and second pressing members may be configured to reduce the distance between them, thereby causing the exertion of pressure to the analysis strip, during the at least part of the movement of the retaining portion from the proximal to the distal position. These first and second pressing members may be configured to increase the distance between them, thereby causing the avoidance of exertion of pressure to the analysis strip, at least when the retaining portion is in any of the proximal and distal positions.

In alternative configurations, other numbers of pressing members (more than two pressing members) may be used for exerting the abovementioned pressure to the strip.

In particular examples, the first and second pressing members may be arranged in such a way that the abovementioned distance reduction causes the exertion of pressure to the analysis strip at corresponding first and second lateral regions of the analysis strip, during the at least part of the movement of the retaining portion from the proximal to the distal position.

Alternatively to having lateral pressing members, the pressing arrangement may be based on at least an upper pressing member and a lower pressing member adapted to apply the pressure to the analysis strip for the retaining piece to cause a suitable retention of the strip.

In even more particular examples, each of the first and second pressing members may comprise a pressing surface by which the first and second pressing members exert the pressure to the analysis strip. Each of these pressing surfaces may be a rough surface for improving the retention of the strip.

In some examples, at least one of the first and second pressing members may be a flexible member configured to be flexed to a compressed position for causing the distance reduction during the at least part of the movement of the retaining portion from the proximal to the distal position, and to be flexed to a relaxed position for causing the distance increase at least when the retaining portion is in any of the proximal and distal positions.

In more particular implementations, both the first and second pressing members may be flexible members configured to be flexed to a compressed position for causing the distance reduction during the at least part of the movement of the retaining portion from the proximal to the distal position, and to be flexed to a relaxed position for causing the distance increase at least when the retaining portion is in any of the proximal and distal positions.

According to some examples, the casing piece may comprise a guiding piece for each flexible pressing member respectively. Besides, each guiding piece may be configured to guide its corresponding flexible pressing member to the compressed position for causing the distance reduction (during the at least part of the movement of the retaining portion from the proximal to the distal position), and to guide its corresponding flexible pressing member to the relaxed position for causing the distance increase (at least when the retaining portion is in any of the proximal and distal positions).

In configurations of the handgrip, the casing piece may be configured to be removably attached with the analyser in a press-fitting manner.

In particular, the casing piece may comprise at least two flanges configured to press against corresponding end sides of the analyser respectively, in such a way that the analyser can be press-fitted within the casing piece. In alternative examples, more than two flanges can be considered and some of them can be configured to press against other sides (e.g. lateral sides) of the analyser.

According to examples of the handgrip, the casing piece may comprise a sheet-shaped portion connecting the at least two flanges and being configured to at least partially cover a rear/bottom side of the analyser when the analyser is press-fitted within the casing piece.

In examples of the handgrip, the retaining piece may comprise a sheet-shaped portion slidably coupled to the sheet-shaped portion of the casing piece. This slidably coupling between the sheet-shaped portion of the retaining piece and the sheet-shaped portion of the casing piece may be based on e.g. a rail based mechanism. An aspect of these examples may be that an operator (e.g. doctor, nurse, etc.) may eject the strip by acting on (touching) the sheet-shaped portion of the retaining piece, which is significantly away from the strip/groove. The risk of infection is thus significantly minimized.

In still more particular examples, the sheet-shaped portion of the retaining piece may have an external rough surface. An aspect of this feature may be that the operator's hand (or fingers) may act on the sheet-shaped portion of the retaining piece in an improved manner.

An analyser may also be provided comprising a groove for inserting an analysis strip and a handgrip configured to extract the analysis strip from the groove. This handgrip may be any of the previously described handgrips. In some examples, this analyser may be provided with the analysis strip inserted in the groove.

Any of the previously commented examples of the handgrip may be made of plastic. More particularly, any of the examples may be made of Acrylonitrile butadiene styrene (ABS). This way, the handgrip may be significantly cheap to manufacture while providing a significant efficiency.

Preferably, in any of the previously described examples, the handgrip may be a removable handgrip, which may be based on e.g. the above mentioned handgrip casing configured to receive the analyser in a press fitting manner.

### BRIEF DESCRIPTION OF THE DRAWINGS

Particular examples of the present invention will be described in the following by way of non-limiting examples, with reference to the appended drawings, in which:
Figure 1 schematically represents a perspective view of a handgrip according to an example, wherein its retaining portion is shown in its distal position;
Figure 2 schematically represents a plan view of a handgrip similar to the one shown in Figure 1, with its retaining portion shown in its distal position;
Figure 3 schematically represents a perspective view of a handgrip similar to the ones shown in Figures 1 and 2, wherein its retaining portion is shown in its proximal position;
Figure 4 schematically represents a plan view of a handgrip similar to the ones shown in Figures 1 - 3, with its retaining portion shown in its proximal position;
Figure 5 schematically represents a lateral view of a handgrip similar to the ones shown in Figures 1 - 4, wherein its retaining portion is in its proximal position;
Figure 6 schematically represents an underside view of a handgrip similar to the ones shown in Figures 1 - 5, with its retaining portion in its proximal position;
Figure 7a schematically represents a sectional view of a front part of a handgrip similar to the ones shown in Figures 1 - 6, wherein its retaining portion is shown in its proximal position;
Figure 7b schematically represents a sectional view of a handgrip's front part similar to the one shown in Figure 7a, with the handgrip's retaining portion shown in an intermediate position between its proximal and distal positions; and
Figure 7c schematically represents a sectional view of a handgrip's front part similar to the ones shown in Figures 7a - 7b, wherein the handgrip's retaining portion is shown in its proximal position.

### DETAILED DESCRIPTION OF EXAMPLES OF THE INVENTION

In the following description, numerous specific details are set forth in order to provide a thorough understanding of examples of the present invention. It will be understood by one skilled in the art however, that examples of the present invention may be practiced without some or all of these specific details. In other instances, well known elements have not been described in detail in order not to unnecessarily obscure the description of the present invention.

In some cases, the same reference numbers have been used to indicate the same or similar elements shown in different Figures.

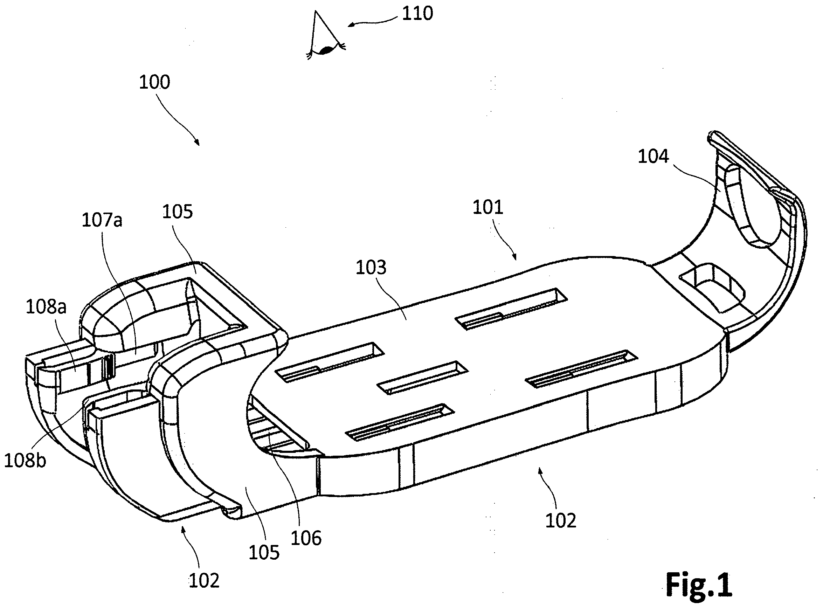

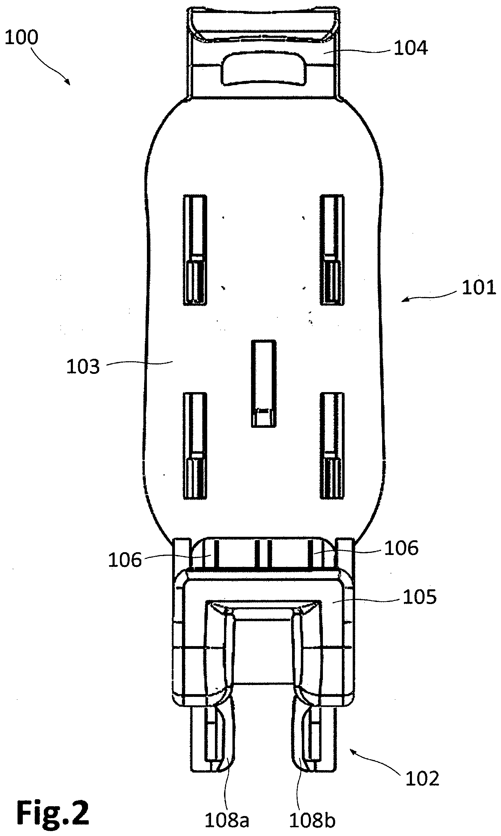

Figure 1 schematically represents a perspective view of a handgrip 100 according to an example. Figure 2 schematically represents a plan view of a handgrip 100 similar to the one shown in Figure 1. The handgrip 100 is shown in these Figures comprising a casing piece 101 and a retaining piece 102, the retaining piece 102 having a retaining portion 108a, 108b being arranged in its distal position in these particular views.

The plan view of Figure 2 may be considered as taken from a top point of vision 110 as indicated in Figure 1.

The casing piece 101 is shown in both Figures comprising two flanges 104, 105 for pressing against corresponding end sides of an analyser to keep the analyser press fitted within the casing piece 101. The flange 104 may have the function of pressing against a rear end of the analyser. The flange 105 may have the function of pressing against a front end of the analyser, said front end having a portion in which a groove for inserting an analysis strip may be located.

In other examples, more than two flanges differently arranged (e.g. for pressing against respective lateral sides of the analyser) may be used to keep the analyser press fitted within the casing piece 101.

The retaining piece 102 may be movable in such a way that the retaining portion 108a, 108b may be movable between a proximal (or receded) position and a distal (or extended) position relative to the front flange 105 of the casing piece 101, i.e. relative to the groove (for inserting an analysis strip) of an analyser when it is mounted within the casing piece. In the particular case of Figures 1 and 2, the retaining portion 108a, 108b is shown in its distal or extended position.

The retaining piece 102 is shown in both Figures comprising a front (retaining) portion compatible with the front flange 105 of the casing piece 101. This front (retaining) portion of the retaining piece 102 may have a first pressing member 108a and a second pressing member 108b both configured to retain (by pressing) the analysis strip during at least part of the movement of the retaining piece 102 between its proximal and distal positions.

These first and second pressing members 108a, 108b may be further adapted to avoid retention of (by not exerting pressure to) the analysis strip at least when the retaining piece 102 is in any of the proximal and distal positions.

The pressing members 108a, 108b may be flexible members such that they can be flexed to a suitable position for laterally pressing (and thus retaining) the strip to be ejected from the analyser. This flexion of the pressing members 108a, 108b may be caused, as commented before, during at least part of the motion of the retaining piece 102 between its proximal and distal positions.

The casing piece 101 may further comprise a guiding element 107a (shown in Figure 1 but not in Figure 2) for guiding the pressing member 108a during the motion of the retaining piece 102 between its proximal and distal positions. The casing piece 101 may further comprise a guiding element (not shown in Figures 1 and 2) for guiding the other pressing member 108b during the motion of the retaining piece 102 between its proximal and distal positions.

More details about the retaining piece 102, its movement between the proximal and distal positions, and how the pressing members 108a, 108b may accordingly behave are provided in other parts of the description with reference to other Figures.

The casing piece 101 may further comprise a sheet-shaped portion 103 connecting the flanges 104, 105, such that a rear/bottom side of an analyser may be at least partially covered by said sheet-shaped portion 103, when the analyser is press-fitted in the casing piece 101.

The retaining piece 102 may also comprise a sheet-shaped portion (not shown in Figures 1 and 2) slidably coupled to the sheet-shaped portion 103 of the casing piece 101. This slidably coupling may be based on a rail based mechanism, a part of which 106 is shown in Figures 1 and 2.

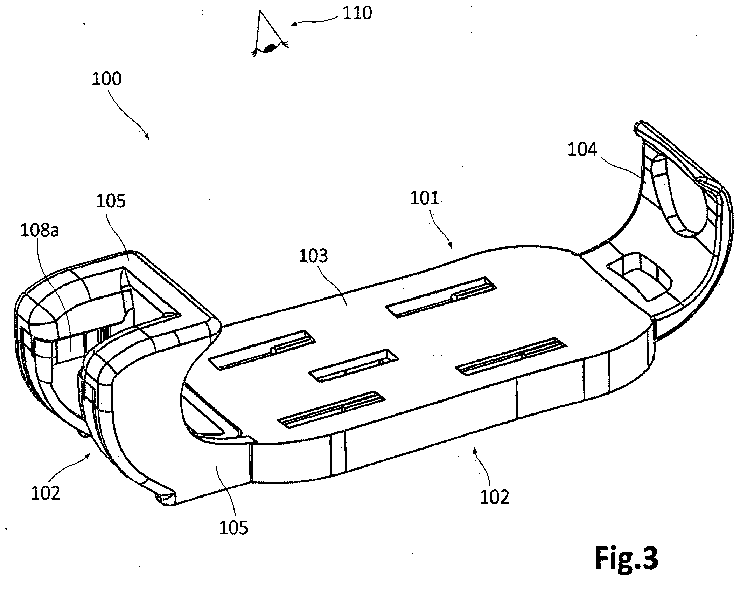

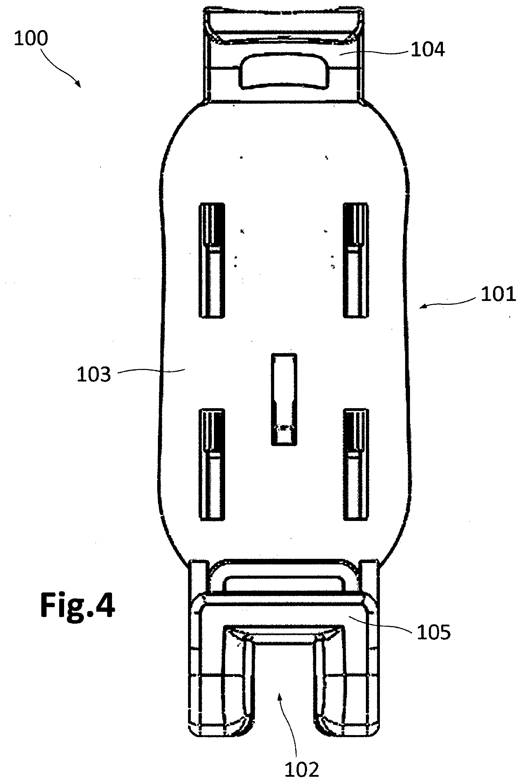

Figure 3 schematically represents a perspective view of a handgrip 100 similar to the ones shown in Figures 1 - 2. Figure 4 schematically represents a plan view of a handgrip 100 similar to the ones shown in Figures 1 - 3. The handgrip 100 is also shown in said Figures 3 and 4 comprising a casing piece 101, and a retaining piece 102 having a retaining portion 108a, 108b arranged in its proximal position in these particular views.

The plan view of Figure 4 may be considered as taken from a top point of vision 110 as indicated in Figure 3.

A front (retaining) portion of the retaining piece 102 (which is relatively clearly shown in Figures 1 and 2) is depicted in Figure 3 almost completely hidden by a front flange 105 of the casing piece 101. In particular, only one pressing member 108a of said front (retaining) portion of the retaining piece 102 is indicated in Figure 3. No part of the retaining portion of the retaining piece 102 is shown in Figure 4, because it appears completely hidden by the front flange 105.

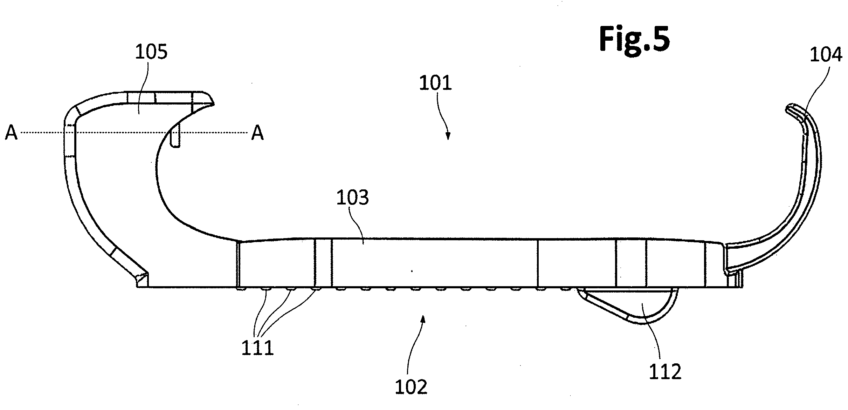

Figure 5 schematically represents a lateral view of a handgrip 100 similar to the ones shown in Figures 1 - 4. The handgrip 100 is also shown in this example having corresponding casing and retaining pieces 101, 102, the retaining piece 102 being in its proximal (or receded) position.

In this Figure, a plurality of small protuberances 111 and a larger protruding piece 112 are shown as forming part of a sheet-shaped portion of the retaining piece 102, which may be slidably coupled to a sheet-shaped portion 103 of the casing piece 101.

This larger protruding piece 112 and/or this plurality of small protuberances 111 may permit an improved contact by an operator's hand (e.g. fingers) on the sheet-shaped portion of the retaining piece 102. This improved contact may facilitate operation of the handgrip 100 by the operator for moving the retaining piece 102 between its proximal and distal positions.

The casing piece 101 is also shown in Figure 5 comprising corresponding rear and front flanges 104, 105 for pressing against respective front and rear ends of an analyser to keep it press fitted within the casing piece 101.

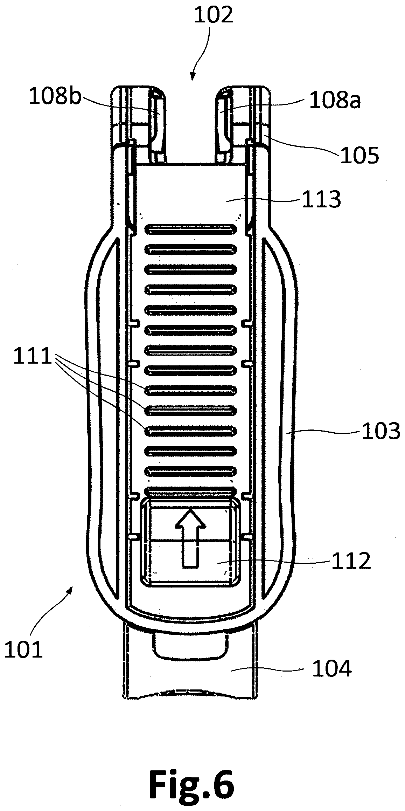

Figure 6 schematically represents an underside view of a handgrip 100 similar to the ones shown in Figures 1 - 5, i.e. from a "bottom" point of vision (not indicated in any of the Figures). The handgrip 100 is shown in this case with its retaining piece 102 in its proximal (or receded) position. This particular view may thus correspond to a handgrip's side opposite to the side shown in Figure 4.

In Figure 6, the retaining piece 102 is shown having a sheet-shaped portion 113 similar to the one described with reference to other Figures. In this case, an outer rough surface with small protruding rows 111 and a larger protruding piece 112 is clearly shown for this sheet-shaped portion 113.

The other elements indicated in Figure 6, such as e.g. front and rear flanges 105, 104 of the casing piece 101, are similarly configured/arranged as described with reference to previous Figures.

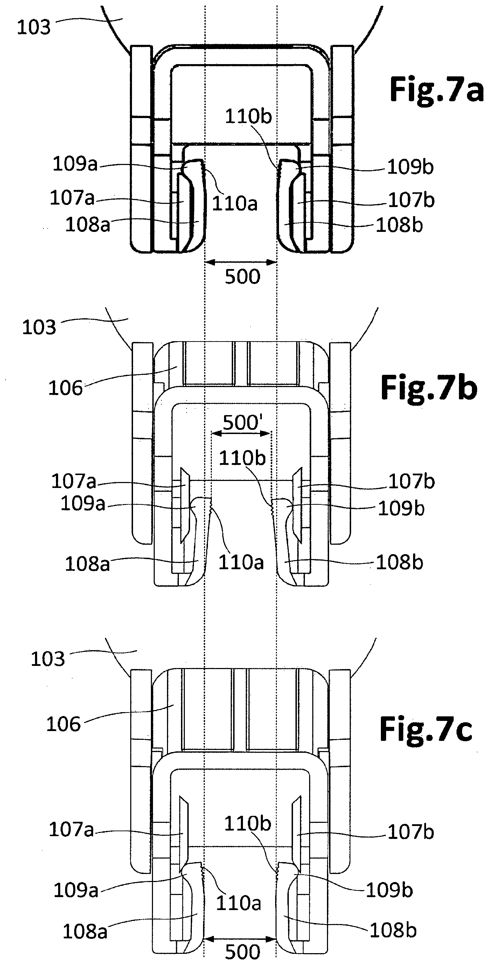

Figures 7a - 7c show respective sectional views of a front part of a handgrip 100 similar to the ones shown in Figures 1 - 6. This sectional view may be considered as taken according to a plane AA indicated in Figure 5.

Each of the Figures 7a - 7c shows first and second pressing members 108a, 108b (of a retaining portion of the handgrip 100) which may be flexible members adapted to be flexed in such a way that they can exert (together) a pressure at corresponding lateral regions of an analysis strip. This pressure that can be exerted to the strip may cause retention of the analysis strip by said pressing members 108a, 108b.

Figure 7b illustrates a particular condition (during at least part of the motion of the retaining portion from its proximal to the distal position) in which said pressure may be caused. Figures 7a and 7c illustrate respective particular conditions (proximal and distal positions of the retaining portion) in which pressure to the strip may be avoided.

As shown in Figures 7a - 7c, each of the (flexible) pressing members 108a, 108b may comprise respective protruding portions 109a, 109b which may suitably interact with corresponding guiding elements 107a, 107b, which are also shown in said Figures 7a - 7c.

Each of the flexible pressing members 108a, 108b may comprise a corresponding pressing surface 110a, 110b by which the pressing members 108a, 108b may exert the aforementioned pressure to the analysis strip (to be removed from an analyser suitably coupled with the handgrip 100).

Three different portions may be distinguished in each of the guiding elements 107a, 107b: a first (inner) recess at one end of the guiding element, a second (outer) recess at the opposite end of the guiding element, and a uniform region between said two (inner and outer) recesses.

The expression "inner recess" may refer herein to that recess of a guiding element 107a, 107b which is always in an innermost position of the handgrip 100 irrespective of the (e.g. proximal, distal, intermediate) position of the retaining piece 102.

The expression "outer recess" may refer herein to that recess of a guiding element 107a, 107b which is always in an outermost position of the handgrip 100 irrespective of the (e.g. proximal, distal, intermediate) position of the retaining piece 102.

As shown in Figure 7a, the protruding region 109a of the pressing member 108a may suitably couple with the inner recess of the guiding element 107a when the retaining piece 102 is in its proximal position. Similarly, the protruding region 109b of the pressing member 108b may also suitably couple with the inner recess of the guiding element 107b when the retaining piece 102 is in its proximal position.

Said coupling of each protruding region 109a, 109b with the inner recess of the corresponding guiding element 107a, 107b may cause the corresponding pressing surfaces 110a, 110b to be separated by a desired distance 500. This distance 500 may be larger than the width of an analysis strip to be ejected, such that no pressure would be exerted by the pressing members 108a, 108b to the analysis strip.

It is worthy of mention that an analysis strip can be easily inserted into a groove of an analyser properly coupled with the handgrip 100, since no opposition is presented by the pressing members 108a, 108b when they are in the position illustrated in Figure 7a.

As shown in Figure 7b, the protruding region 109a of the pressing member 108a may suitably contact the uniform region (between the inner and outer recesses) of the guiding element 107a when the retaining piece 102 is in an intermediate position (between the proximal and distal positions) of the retaining piece 102.

Similarly, the protruding region 109b of the pressing member 108b may suitably contact the uniform region (between the inner and outer recesses) of the guiding element 107b when the retaining piece 102 is in an intermediate position (between the proximal and distal positions) of the retaining piece 102.

Said contact of each protruding region 109a, 109b with the uniform region (between the inner and outer recesses) of the corresponding guiding element 107a, 107b may cause the corresponding pressing surfaces 110a, 110b to be separated by a desired distance 500'. This distance 500' may be smaller than the distance 500 (see Figures 7a and 7c) and of an amount such that a certain pressure may be exerted by the pressing members 108a, 108b to the analysis strip.

This pressure may thus be continuously exerted to the strip along the entire length of the uniform regions (between the inner and outer recesses) of both guiding elements 107a, 107b. This continuous pressure may cause the pressing members 108a, 108b to retain (i.e. pull) the strip until it is extracted from a corresponding analyser's groove.

As shown in Figure 7c, the protruding region 109a of the pressing member 108a may suitably couple with the outer recess of the guiding element 107a when the retaining piece 102 is in its distal position. Similarly, the protruding region 109b of the pressing member 108b may also suitably couple with the outer recess of the guiding element 107b when the retaining piece 102 is in its distal position.

Said coupling of each protruding region 109a, 109b with the outer recess of the corresponding guiding element 107a, 107b may cause the corresponding pressing surfaces 110a, 110b to be separated by a desired distance 500. This distance 500 may be larger than the width of an analysis strip to be ejected, such that no pressure would be exerted by the pressing members 108a, 108b to the analysis strip.

The abovementioned continuous pressure exerted by the pressing members 108a, 108b to the strip (as described with reference to Figure 7b) may be ceased when the pressing members 108a, 108b achieve the condition of Figure 7c. This way, the strip can be finally released as a result of said cessation of the pressure.

In some or all of the described examples, the handgrip 100 may be manufactured either separated from the analyser or integrated with the analyser. In the former case, the handgrip 100 may be a removable handgrip, such that it may be provided individually. On the contrary, in the latter case, the analyser may be provided with the handgrip 100 as a single piece. Thus, for example, a side of the analyser may coincide with the casing piece of the handgrip.

An aspect of providing a removable handgrip may be that a damaged handgrip may be easily replaced by another in a very easy and cheap manner. Another aspect of providing a removable handgrip may be that a same handgrip may be reused, applied to different analysers of the same type/shape/size. Of course, different sizes/ shapes for the removable handgrip may be taken into account depending on the model of analyser with which the handgrip is intended to be used.

Although only a number of particular examples of the invention have been disclosed herein, it will be understood by those skilled in the art that other alternative examples and/or uses of the invention and obvious modifications and equivalents thereof are possible. Furthermore, the present invention covers all possible combinations of the particular examples described. Thus, the scope of the present invention should not be limited by particular examples, but should be determined only by a fair reading of the claims that follow.

## Claims

1. A handgrip (100) configured to extract an analysis strip from a groove of an analyser,
the handgrip comprising:
a casing piece (101) configured to be attached with the analyser; and
a retaining piece (102) having a retaining portion, (108a, 108b) the retaining piece being movable with respect to the casing piece in such a way that the retaining portion is movable in a first direction from a proximal position to a distal position relative to the casing piece; **characterized in that**:
the retaining portion is configured to retain the analysis strip during at least part of its movement from the proximal to the distal position, and to avoid retaining the analysis strip at least when the retaining portion is in any of the proximal and distal positions.

2. A handgrip according to claim 1, wherein:
the retaining portion comprises a pressing arrangement; (108a, 108b) the pressing arrangement is configured to exert pressure to the analysis strip, thereby causing the retention of the analysis strip, during the at least part of the movement of the retaining portion from the proximal to the distal position; and
the pressing arrangement is configured to avoid exerting pressure to the analysis strip, thereby causing the avoidance of retention of the analysis strip, at least when the retaining portion is in any of the proximal and distal positions.

3. A handgrip according to claim 2, wherein:
the pressing arrangement comprises a first and a second pressing member (108a, 108b) facing each other and defining a path having a distance between them, said path being perpendicular to the first direction;
the first and second pressing members are configured to reduce the distance between them, thereby causing the exertion of pressure to the analysis strip, during the at least part of the movement of the retaining portion from the proximal to the distal position; and
the first and second pressing members are configured to increase the distance between them, thereby causing the avoidance of exertion of pressure to the analysis strip, at least when the retaining portion is in any of the proximal and distal positions.

4. A handgrip according to claim 3, wherein the first and second pressing members are arranged in such a way that the distance reduction causes the exertion of pressure to the analysis strip at corresponding first and second lateral regions of the analysis strip, during the at least part of the movement of the retaining portion from the proximal to the distal position.

5. A handgrip according to any of claims 3 or 4, wherein at least one of the first and second pressing members is a flexible member configured to be flexed to a compressed position for causing the distance reduction during the at least part of the movement of the retaining portion from the proximal to the distal position, and to be flexed to a relaxed position for causing the distance increase at least when the retaining portion is in any of the proximal and distal positions.

6. A handgrip according to claim 5, wherein both the first and second pressing members are flexible members configured to be flexed to the compressed position for causing the distance reduction during the at least part of the movement of the retaining portion from the proximal to the distal position, and to be flexed to the relaxed.position for causing the distance increase at least when the retaining portion is in any of the proximal and distal positions.

7. A handgrip according to any of claims 5 or 6, wherein the casing piece comprises a guiding piece (107a) for each flexible pressing member respectively, each guiding piece being configured to guide its corresponding flexible pressing member to the compressed position for causing the distance reduction during the at least part of the movement of the retaining portion from the proximal to the distal position, and to guide its corresponding flexible pressing member to the relaxed position for causing the distance increase at least when the retaining portion is in any of the proximal and distal positions.

8. A handgrip according to any of claims 1 to 7, wherein the casing piece is configured to be removably attached with the analyser in a press-fitting manner.

9. A handgrip according to claim 8, wherein the casing piece comprises at least two flanges (104, 105) configured to press against corresponding end sides of the analyser respectively, in such a way that the analyser can be press-fitted within the casing piece.

10. A handgrip according to claim 9, wherein the casing piece comprises a sheet-shaped portion (103) connecting the at least two flanges and being configured to at least partially cover a rear/bottom side of the analyser when the analyser is press-fitted within the casing piece.

11. A handgrip according to claim 10, wherein the retaining piece comprises a sheet-shaped portion slidably coupled to the sheet-shaped portion of the casing piece.

12. A handgrip according to claim 11, further comprising a rail based mechanism; wherein the sheet-shaped portion of the retaining piece is slidably coupled with the sheet-shaped portion of the casing piece by said rail based mechanism.

13. A handgrip according to any of claims 11 or 12, wherein the sheet-shaped portion of the retaining piece has an external rough surface.

14. An analyser comprising:
a groove for inserting an analysis strip; and
a handgrip configured to extract the analysis strip from the groove, according to any of claims 1 to 13.

15. An analyser according to claim 14, further comprising the analysis strip inserted in the groove.

## Patentansprüche

1. Handgriff (100), der dazu ausgelegt ist, einen Analysestreifen aus einem Schlitz eines Analysegeräts herauszuziehen, wobei der Handgriff Folgendes umfasst:
ein Gehäuseteil (101), das zur Verbindung mit dem Analysegerät ausgelegt ist; und
ein Halteteil (102) mit einem Halteabschnitt (108a, 108b), wobei das Halteteil bezüglich des Gehäuseteils beweglich ist, derart, dass der Halteabschnitt in eine erste Richtung aus einer proximalen Position in eine distale Position bezüglich des Gehäuseteils beweglich ist;
**dadurch gekennzeichnet, dass**:
der Halteabschnitt dazu ausgelegt ist, den Analysestreifen zumindest während eines Teils seiner Bewegung aus der proximalen in die distale Position festzuhalten, und Festhalten des Analysestreifens zumindest dann zu vermeiden, wenn sich der Halteabschnitt in der proximalen oder der distalen Position befindet.

2. Handgriff nach Anspruch 1, wobei:
der Halteabschnitt eine Druckanordnung umfasst (108a, 108b);
wobei die Druckanordnung dazu ausgelegt ist, zumindest während des Teils der Bewegung des Halteabschnitts aus der proximalen in die distale Position den Analysestreifen mit Druck zu beaufschlagen, wodurch der Analysestreifen festgehalten wird; und
die Druckanordnung dazu ausgelegt ist, Druckbeaufschlagung des Analysestreifens zu vermeiden, wodurch Festhalten des Analysestreifens vermieden wird, zumindest dann, wenn sich der Halteabschnitt in der proximalen oder der distalen Position befindet.

3. Handgriff nach Anspruch 2, wobei:
die Druckanordnung ein erstes und ein zweites Druckelement (108a, 108b) umfasst, die einander zugewandt sind und einen Weg mit einem Abstand dazwischen definieren, wobei der Weg senkrecht zur ersten Richtung ist;
das erste und das zweite Druckelement dazu ausgelegt sind, den Abstand zwischen sich zu verringern, wodurch die Druckbeaufschlagung des Analysestreifens verursacht wird, zumindest während des Teils der Bewegung des Halteabschnitts aus der proximalen in die distale Position; und
das erste und das zweite Druckelement dazu ausgelegt sind, den Abstand zwischen sich zu vergrößern, wodurch die Druckbeaufschlagung des Analysestreifens vermieden wird, zumindest dann, wenn sich der Halteabschnitt in der proximalen oder der distalen Position befindet.

4. Handgriff nach Anspruch 3, wobei das erste und das zweite Druckelement auf eine solche Weise angeordnet sind, dass die Verringerung des Abstands die Druckbeaufschlagung des Analysestreifens an entsprechenden ersten und zweiten Seitenregionen des Analysestreifens verursacht, zumindest während des Teils der Bewegung des Halteabschnitts aus der proximalen in die distale Position.

5. Handgriff nach einem der Ansprüche 3 oder 4, wobei zumindest eines des ersten und des zweiten Druckelements ein flexibles Element ist, das dazu ausgelegt ist, in eine komprimierte Position gebogen zu werden, um die Verringerung des Abstands zumindest während des Teils der Bewegung des Halteabschnitts aus der proximalen in die distale Position zu verursachen, und in eine entspannte Position gebogen zu werden, um die Vergrößerung des Abstands zumindest dann zu verursachen, wenn sich der Halteabschnitt in der proximalen oder der distalen Position befindet.

6. Handgriff nach Anspruch 5, wobei sowohl das erste als auch das zweite Druckelement flexible Elemente sind, die dazu ausgelegt sind, in die komprimierte Position gebogen zu werden, um die Verringerung des Abstands zumindest während des Teils der Bewegung des Halteabschnitts aus der proximalen in die distale Position zu verursachen, und in die entspannte Position gebogen zu werden, um die Vergrößerung des Abstands zumindest dann zu verursachen, wenn sich der Halteabschnitt in der proximalen oder der distalen Position befindet.

7. Handgriff nach einem der Ansprüche 5 oder 6, wobei das Gehäuseteil jeweils ein Führungsteil (107a) für jedes flexible Druckelement umfasst, wobei jedes Führungsteil dazu ausgelegt ist, sein entsprechendes flexibles Druckelement in die komprimierte Position zu führen, um die Verringerung des Abstands zumindest während des Teils der Bewegung des Halteabschnitts aus der proximalen in die distale Position zu verursachen, und sein entsprechendes flexibles Druckelement in die entspannte Position zu führen, um die Vergrößerung des Abstands zumindest dann zu verursachen, wenn sich der Halteabschnitt in der proximalen oder der distalen Position befindet.

8. Handgriff nach einem der Ansprüche 1 bis 7, wobei das Gehäuseteil dazu ausgelegt ist, durch Presspassung lösbar mit dem Analysegerät befestigt zu werden.

9. Handgriff nach Anspruch 8, wobei das Gehäuseteil zumindest zwei Flansche (104, 105) umfasst, die dazu ausgelegt sind, jeweils gegen entsprechende Endseiten des Analysegeräts zu drücken, derart, dass das Analysegerät durch Druck in das Gehäuseteil eingepasst werden kann.

10. Handgriff nach Anspruch 9, wobei das Gehäuseteil einen flächenförmigen Abschnitt (103) umfasst, der die mindestens zwei Flansche miteinander verbindet und dazu ausgelegt ist, eine Rückseite/Bodenseite des Analysegeräts zumindest teilweise abzudecken, wenn das Analysegerät durch Druck in das Gehäuseteil eingepasst wird.

11. Handgriff nach Anspruch 10, wobei das Halteteil einen flächenförmigen Abschnitt umfasst, der verschiebbar mit dem flächenförmigen Abschnitt des Gehäuseteils verbunden ist.

12. Handgriff nach Anspruch 11, ferner umfassend einen Mechanismus auf Schienenbasis; wobei der flächenförmige Abschnitt des Halteteils über den Mechanismus auf Schienenbasis verschiebbar mit dem flächenförmigen Abschnitt des Gehäuseteils verbunden ist.

13. Handgriff nach einem der Ansprüche 11 oder 12, wobei der flächenförmige Abschnitt des Halteteils eine raue Oberfläche aufweist.

14. Analysegerät, umfassend:
einen Schlitz zur Einführung eines Analysestreifens; und
einen Handgriff, der dazu ausgelegt ist, den Analysestreifen aus dem Schlitz herauszuziehen, nach einem der Ansprüche 1 bis 13.

15. Analysegerät nach Anspruch 14, ferner umfassend den in den Schlitz eingeführten Analysestreifen.

## Revendications

1. Poignée (100) conçue pour extraire une bande d'analyse d'une rainure d'un analyseur, la poignée comprenant :
une pièce d'enveloppe (101) conçue pour être attachée à l'analyseur ; et
une pièce de maintien (102) ayant une portion de maintien (108a, 108b), la pièce de maintien étant mobile par rapport à la pièce d'enveloppe d'une manière telle que la portion de maintien soit mobile dans une première direction depuis une position proximale jusqu'à une position distale par rapport à la pièce d'enveloppe ;
**caractérisée en ce que** :
la portion de maintien est conçue pour maintenir la bande d'analyse durant au moins une partie de son déplacement depuis la position proximale jusqu'à la position distale, et pour éviter le maintien de la bande d'analyse au moins lorsque la portion de maintien se trouve dans l'une quelconque des positions proximale et distale.

2. Poignée selon la revendication 1, dans laquelle :
la portion de maintien comprend un agencement de pression (108a, 108b) ;
l'agencement de pression est conçu pour exercer une pression sur la bande d'analyse, provoquant de ce fait le maintien de la bande d'analyse, durant cette au moins une partie du déplacement de la portion de maintien depuis la position proximale jusqu'à la position distale ; et
l'agencement de pression est conçu pour éviter d'exercer une pression sur la bande d'analyse, provoquant de ce fait l'évitement du maintien de la bande d'analyse, au moins lorsque la portion de maintien se trouve dans l'une quelconque des positions proximale et distale.

3. Poignée selon la revendication 2, dans laquelle :
l'agencement de pression comprend un premier et un deuxième organe de pression (108a, 108b) se faisant face et définissant un trajet présentant une distance entre eux, ledit trajet étant perpendiculaire à la première direction ;
les premier et deuxième organes de pression sont conçus pour réduire la distance entre eux, provoquant de ce fait l'exercice d'une pression sur la bande d'analyse, durant cette au moins une partie du déplacement de la portion de maintien depuis la position proximale jusqu'à la position distale ; et
les premier et deuxième organes de pression sont conçus pour augmenter la distance entre eux, provoquant de ce fait l'évitement de l'exercice d'une pression sur la bande d'analyse, au moins lorsque la portion de maintien se trouve dans l'une quelconque des positions proximale et distale.

4. Poignée selon la revendication 3, dans laquelle les premier et deuxième organes de pression sont disposés d'une manière telle que la réduction de distance provoque l'exercice d'une pression sur la bande d'analyse en des première et deuxième régions latérales correspondantes de la bande d'analyse, durant cette au moins une partie du déplacement de la portion de maintien depuis la position proximale jusqu'à la position distale.

5. Poignée selon l'une quelconque des revendications 3 ou 4, dans laquelle au moins l'un des premier et deuxième organes de pression est un organe souple conçu pour être fléchi jusqu'à une position comprimée pour provoquer la réduction de distance durant cette au moins une partie du déplacement de la portion de maintien depuis la position proximale jusqu'à la position distale, et pour être fléchi jusqu'à une position relâchée pour provoquer l'augmentation de distance au moins lorsque la portion de maintien se trouve dans l'une quelconque des positions proximale et distale.

6. Poignée selon la revendication 5, dans laquelle les premier et deuxième organes de pression sont tous les deux des organes souples conçus pour être fléchis jusqu'à la position comprimée pour provoquer la réduction de distance durant cette au moins une partie du déplacement de la portion de maintien depuis la position proximale jusqu'à la position distale, et pour être fléchis jusqu'à la position relâchée pour provoquer l'augmentation de distance au moins lorsque la portion de maintien se trouve dans l'une quelconque des positions proximale et distale.

7. Poignée selon l'une quelconque des revendications 5 ou 6, dans laquelle la pièce d'enveloppe comprend une pièce de guidage (107a) pour chaque organe de pression souple respectivement, chaque pièce de guidage étant conçue pour guider son organe de pression souple correspondant jusqu'à la position comprimée pour provoquer la réduction de distance durant cette au moins une partie du déplacement de la portion de maintien depuis la position proximale jusqu'à la position distale, et pour guider son organe de pression souple correspondant jusqu'à la position relâchée pour provoquer l'augmentation de distance au moins lorsque la portion de maintien se trouve dans l'une quelconque des positions proximale et distale.

8. Poignée selon l'une quelconque des revendications 1 à 7, dans laquelle la pièce d'enveloppe est conçue pour être attachée amovible à l'analyseur par ajustement par pression.

9. Poignée selon la revendication 8, dans laquelle la pièce d'enveloppe comprend au moins deux brides (104, 105) conçues pour se presser contre des côtés d'extrémité correspondants de l'analyseur respectivement, d'une manière telle que l'analyseur puisse être ajusté par pression au sein de la pièce d'enveloppe.

10. Poignée selon la revendication 9, dans laquelle la pièce d'enveloppe comprend une portion en forme de feuille (103) reliant ces au moins deux brides et étant conçue pour recouvrir au moins partiellement un côté arrière/inférieur de l'analyseur lorsque l'analyseur est ajusté par pression au sein de la pièce d'enveloppe.

11. Poignée selon la revendication 10, dans laquelle la pièce de maintien comprend une portion en forme de feuille accouplée coulissante à la portion en forme de feuille de la pièce d'enveloppe.

12. Poignée selon la revendication 11, comprenant en outre un mécanisme à base de rail ; dans laquelle la portion en forme de feuille de la pièce de maintien est accouplée coulissante à la portion en forme de feuille de la pièce d'enveloppe par ledit mécanisme à base de rail.

13. Poignée selon l'une quelconque des revendications 11 ou 12, dans laquelle la portion en forme de feuille de la pièce de maintien présente une surface externe rugueuse.

14. Analyseur comprenant :
une rainure pour insérer une bande d'analyse ; et
une poignée conçue pour extraire la bande d'analyse de la rainure, selon l'une quelconque des revendications 1 à 13.

15. Analyseur selon la revendication 14, comprenant en outre la bande d'analyse insérée dans la rainure.
